# FASCICULE DE BREVET EUROPEEN

(11) **EP 4 190 388 B1**
(45) Date de publication et mention de la délivrance du brevet: **17.09.2025**
(21) Numéro de dépôt: 22211149.4
(22) Date de dépôt: 02.12.2022
(51) Int. Cl.: A61M 39/28, A61M 5/168

(54) **RÉDUCTEUR DE DÉBIT POUR TUBE SOUPLE**
STRÖMUNGSREDUZIERER FÜR EIN FLEXIBLES ROHR
FLOW RESTRICTOR FOR FLEXIBLE TUBE

(30) Priorité: 03.12.2021 FR 2112929
(43) Date de publication de la demande: 07.06.2023
(73) Titulaire: Maco Pharma, 59420 Mouvaux (FR)
(72) Inventeur: Coasne, Nicolas, Mouvaux (FR); Brebant, Quentin, 59370 Mons en Baroeul (FR); Nicholson, Gordon, Worcester, WR2 6LL (GB)
(74) Mandataire: Sayettat, Julien Christian

(56) Documents cités:
- US-A- 4 248 401
- US-A- 4 434 963

## Description

La présente invention concerne un système comprenant un tube souple et une pince pour réduire le débit d'un fluide dans ledit tube souple, ainsi qu'un dispositif pour éliminer les leucocytes du sang ou d'un composant sanguin comprenant une telle pince.

L'invention s'applique au domaine des dispositifs médicaux et notamment aux dispositifs destinés à la filtration du sang ou d'un composant sanguin.

Le sang total est constitué de deux types de composants : les cellules sanguines comprenant les globules rouges, les leucocytes et les plaquettes, et le plasma, liquide jaune pâle dans lequel les cellules sanguines sont en suspension.

Il s'est avéré que les leucocytes ont des effets indésirables très importants, ce qui a conduit à chercher à les éliminer des composants sanguins destinés à la transfusion. En effet, les leucocytes augmentent notamment le risque de rejet immunitaire tel que la maladie du greffon contre l'hôte et favorisent la transmission d'agents infectieux.

Pour éliminer les leucocytes des composants sanguins destinés à la transfusion, il existe sur le marché des unités de filtration renfermant un milieu de déleucocytation comprenant généralement un assemblage de plusieurs couches de fibres non-tissés de polyester. Cet assemblage retient sélectivement les leucocytes du sang par deux mécanismes principaux de rétention, à savoir le tamisage et l'adhésion.

La performance de rétention des leucocytes de ces unités de filtration dépend de nombreux paramètres non seulement liés à des caractéristiques intrinsèques des unités de filtration telles que la surface de filtration, le diamètre des fibres, la densité de fibres, le temps de passage dans le filtre, mais aussi liés à des caractéristiques extrinsèques telles que la composition du sang à filtrer, la durée et la température de stockage du sang avant filtration ou la température du sang au moment de sa filtration.

Notamment, il est connu qu'une filtration du sang à haute température, c'est-à-dire supérieure à 24°C entraîne une hausse du taux de leucocytes résiduels par rapport à une filtration à température ambiante ou à 4°C.

Une explication possible est que plus la température du sang augmente, plus sa viscosité du sang diminue. Ainsi, au-delà de 24°C l'écoulement du sang dans l'unité de filtration sera plus rapide et le temps de contact des leucocytes avec le milieu de déleucocytation réduit. En outre, les leucocytes deviennent également plus déformables lorsque la température augmente, rendant moins efficace l'action de tamisage du milieu de déleucocytation.

Afin de maintenir le débit du sang entre 20 et 50 ml/min dans un dispositif pour éliminer les leucocytes du sang ou d'un composant sanguin de faible viscosité, le document US 6,013,184 propose un dispositif comprenant un port d'entrée, un filtre pour éliminer les leucocytes, un port de sortie et une tubulure qui connecte, dans cet ordre, le port d'entrée, le filtre pour éliminer les leucocytes et le port de sortie. Une portion étroite destinée à réguler le débit du sang dans ledit dispositif est prévue dans ladite tubulure souple, du côté aval du filtre. La portion étroite de la tubulure souple est réalisée soit en insérant un tube creux ayant une section transversale utile spécifiquement réduite dans ladite tubulure souple, soit en déformant par compression la tubulure souple à l'aide d'une pince coulissante. Les pinces coulissantes doivent être conçues pour que la portion étroite de la tubulure souple possède une section transversale utile comprise entre 0,1 mm² et 5,0 m² et un rapport de la longueur à la section transversale utile comprise entre 3 à 400 mm /mm2. Dans les exemples, la longueur de la portion étroite dans la tubulure souple est ainsi comprise entre 25 et 240 mm, ce qui rendrait peu maniable une pince avec de telles dimensions.

On connaît également du document DE 36 31 411 une pince pour modifier la section d'écoulement d'un tube souple. Cette pince comprend une pluralité d'ouvertures de largeur différente, reliées les unes aux autres, permettant d'obtenir des sections d'écoulement de différentes tailles.

Le document US 4,434,963 divulgue quant à lui une pince coulissante de forme généralement plate servant à obturer un tube souple. Cette pince n'est pas conçue pour réduire le débit de fluide dans un tube souple mais pour stopper complètement l'écoulement du fluide dans ledit tube souple.

Le document US 4,248,401 divulgue le même type de pince coulissante que le document précédent, prévue pour stopper l'écoulement du fluide dans un tube souple. Cette pince présente une fente en forme de larme destinée à recevoir et obturer le tube souple. Un insert ayant des surfaces de pincement parallèles entre elles et perpendiculaires au plan de la pince est placé dans cette fente.

L'invention propose de réduire facilement le débit d'un fluide dans un tube souple à l'aide d'une pince améliorée. En particulier, cette pince arrangée dans un dispositif pour éliminer les leucocytes du sang ou d'un composant sanguin permet d'obtenir un taux de déleucocytation conformes aux normes européennes lorsque le sang à filtrer possède une température allant de 24°C à 30°C.

A cet effet et selon un premier aspect, l'invention propose un système pour réduire le débit d'un fluide dans un tube souple comprenant ledit tube souple et une pince, ladite la pince comprenant un corps pourvu d'une ouverture destinée à recevoir ledit tube souple, l'ouverture ayant au moins une portion de pincement configurée pour comprimer ledit tube souple sans l'obturer, ladite portion de pincement est définie entre une première surface de pincement et une deuxième surface de pincement qui se font face et qui se rapprochent l'une de l'autre sur une hauteur plus petite ou égale à la largeur du tube souple lorsque ledit tube souple est comprimé jusqu'à obturation, de sorte à réduire le débit de fluide dans ledit tube souple.

Selon un deuxième aspect, l'invention concerne un dispositif pour éliminer les leucocytes du sang ou d'un composant sanguin, comprenant d'une part un filtre pour éliminer les leucocytes du sang ou d'un composant sanguin et un premier tube souple en communication fluidique avec ledit filtre et d'autre part une pince placée ou destinée à être placée sur le premier tube souple afin de réduire le débit dans ledit premier tube souple.

Les dessins annexés illustrent l'invention :
[Fig.1] représente une vue en perspective et de dessus d'une pince selon un premier mode de réalisation de l'invention.
[Fig.2] représente une vue en coupe de la pince de la figure 1 avec un tube souple y inséré.
[Fig.3] représente une vue en perspective et de dessus d'une pince selon un deuxième mode de réalisation de l'invention.
[Fig.4] représente une vue de face de la pince de la figure 2.
[Fig.5] représente une vue en perspective et de dessus d'une pince selon un troisième mode de réalisation de l'invention.
[Fig.6] représente une vue en perspective et de dessous de la pince de la figure 5.
[Fig.7] représente une vue en coupe de la pince de la figure 5.
[Fig.8] représente une vue en coupe d'une variante de la pince de la figure 5.
[Fig.9] représente une vue en coupe d'une autre variante de la pince de la figure 5.
[Fig.10] représente une vue schématique d'un dispositif pour éliminer les leucocytes comprenant une pince selon l'invention.

Dans la description qui suit, les termes "supérieur" et "inférieur" sont définis par rapport au sens d'utilisation de la pince lorsqu'elle est placée sur un tube souple vertical.

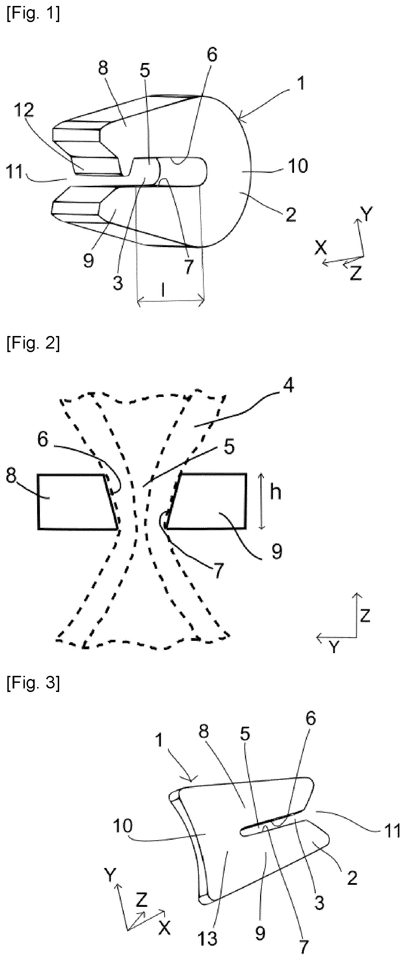

La figure 1 représente une pince pour réduire le débit de fluide dans un tube souple selon un premier mode de réalisation.

Cette pince est destinée à comprimer un tube souple, sans l'obturer complètement. Le fluide s'écoule dans le tube souple, y compris au travers de la partie comprimée par la pince. La compression réduit la section transversale du tube souple, réduisant ainsi le débit de fluide dans le tube souple par rapport au tube souple non comprimé.

Selon une première réalisation des figures 1 et 2, la pince 1 comprend un corps 2 pourvu d'une ouverture 3 destinée à recevoir un tube souple 4. L'ouverture 3 possède au moins une portion de pincement 5 configurée pour comprimer ledit tube souple 4 sans l'obturer. La portion de pincement 5 est définie entre une première surface de pincement 6 et une deuxième surface de pincement 7 qui se font face et qui se rapprochent l'une de l'autre sur une hauteur h plus petite ou égale à la largeur du tube souple 4 lorsque ledit tube souple est comprimé jusqu'à obturation, de sorte à réduire le débit de fluide dans ledit tube souple.

Selon la figure 1, le corps 2 de la pince 1 est substantiellement plate. En outre, le corps de la pince présente substantiellement une forme en U. Dans la description qui suit et selon l'espace (x,y,z) référencé sur la figure 1, la longueur de la pince 1 se trouve dans l'axe x, la largeur dans l'axe y et la hauteur ou l'épaisseur dans l'axe z.

Le corps 2 de la pince comprend un premier bras 8 et un deuxième bras 9 dans le sens de la longueur, lesdits premier et deuxième bras 8,9 étant connectés entre eux par une base 10. Le premier et le deuxième bras 8,9 comprennent la première et deuxième surface de pincement 6,7, respectivement. L'ouverture 3 de la pince se situe entre le premier et le deuxième bras 8,9.

Sur la figure 1, l'ouverture 3 de la pince comprend en outre une portion d'insertion 11 configurée pour introduire le tube souple 4 à comprimer dans l'ouverture 3, ladite portion d'insertion 11 étant en communication avec la portion de pincement 5.

La portion d'insertion 11 est formée aux extrémités du premier bras 8 et du deuxième bras 9 du corps 2. La distance entre le premier et deuxième bras 8,9 au niveau de la portion d'insertion 11 est plus grande que celle au niveau de la portion de pincement 5. Vue de dessus, la portion d'insertion 11 possède substantiellement une forme en V. Ainsi, la réduction de la section transversale du tube est progressive dans le sens de l'écoulement du fluide dans le tube.

Selon la figure 1, l'un des premier ou deuxième bras 8,9 possède une saillie 12 au niveau de la portion d'insertion 11 configurée pour maintenir le tube souple 4 dans l'ouverture 3 du corps 2 de la pince et notamment dans la portion de pincement 5.

Selon la réalisation de la figure 1, la première et la deuxième surface de pincement 6,7 sont substantiellement planes.

Les surfaces de pincement 6,7 de la pince se rapprochent l'une de l'autre dans le sens de la hauteur, c'est-à-dire la distance entre lesdites surfaces de pincement 6,7 est plus petite au niveau de la face inférieure de la pince qu'au niveau de la face supérieure. Ainsi, vue en coupe, les surfaces de pincement 6,7 forment un V et la section transversale du tube souple inséré dans la pince 1 se réduit dans le sens de l'écoulement du fluide (figure 2).

De plus, afin de faciliter le maniement de la pince, la hauteur h de la portion de pincement 5 est plus petite ou égale à la largeur du tube souple 4 lorsque ledit tube souple est comprimé jusqu'à obturation.

En outre, la longueur l de la surface de pincement est supérieure ou égale à la largeur L du tube souple 4 lorsque ledit tube souple est comprimé jusqu'à obturation afin d'engager complètement le tube souple 4 dans la portion de pincement 5.

Par exemple, lorsque le tube souple possède un diamètre intérieur de 3 mm et un diamètre extérieur de 4,1 mm, la largeur du tube souple lorsqu'il est comprimé jusqu'à obturation est d'environ 5,1 mm. La portion de pincement possède par exemple une largeur d'environ 1,5 mm au niveau de la face supérieure et une largeur d'environ 1,3 mm au niveau de la face inférieure. La hauteur de la portion de pincement est d'environ 5 mm. La longueur de la portion de pincement est d'environ 10 mm.

La pince 1 pour réduire le débit d'un fluide dans un tube souple est suffisamment rigide pour comprimer le tube souple sans que la pince se déforme. Cette pince est réalisée par impression 3D ou par moulage par injection. Des exemples de matériau pour réaliser la pince sont le polypropylène, le polycarbonate, le polyamide ou un copolymère d'acrylate.

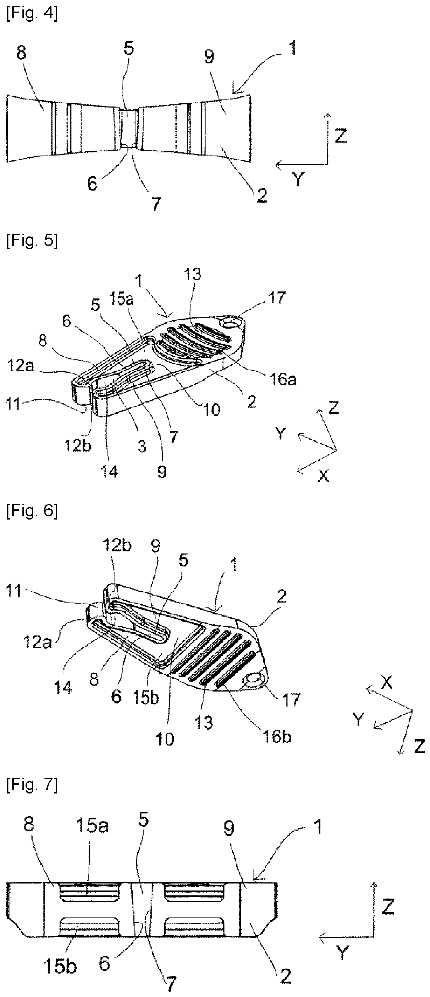

Les figures 3 et 4 représentent une deuxième réalisation d'une pince selon l'invention. Cette deuxième réalisation de différencie de la première réalisation en ce que la pince 1 comprend une partie de préhension 13 permettant à l'utilisateur de se saisir facilement de la pince. Dans cette deuxième réalisation, la partie de préhension 13 est essentiellement constituée de la base 10 du corps 2 de la pince, celle-ci ayant des dimensions conçues pour que l'utilisateur puisse placer ses doigts sur la surface supérieure et la surface inférieure de ladite base 10 du corps de la pince.

En outre, le bord latéral de la partie de préhension 13 est concave afin de faciliter l'insertion du tube souple 4 dans ladite pince 2, l'utilisateur poussant la pince 2 au niveau de cette concavité à l'aide d'un de ses doigts ou du pouce pour insérer le tube souple 4 dans ladite pince 2.

Dans cette deuxième réalisation, ni le premier bras 8 ni le deuxième bras 9 du corps 2 de la pince ne comprend de saillie.

Les figues 5 à 9 représentent une troisième réalisation d'une pince selon l'invention.

Cette troisième réalisation se différencie de la première réalisation de la pince en ce que l'ouverture 3 de la pince comprend en outre une portion de maintien 14 dans laquelle le tube souple 4 est maintenu sans être déformé ou comprimé. La largeur de la portion de maintien 14 est substantiellement égale au diamètre extérieur du tube souple. Cette portion de maintien permet d'associer la pince à un tube souple, sans le comprimer.

Comme illustré sur les figures 5 et 6, la portion de maintien 14 est en communication avec la portion de pincement 5 de l'ouverture 3.

La portion de maintien 14 est en outre en communication avec la portion d'insertion 11 de l'ouverture 3 de la pince. En insérant le tube souple 4 dans la pince, celui-ci pénètre d'abord dans la portion d'insertion 11, puis la portion de maintien 14 et enfin la portion de pincement 5.

Dans cette troisième réalisation, chacun du premier bras 8 et du deuxième bras 9 comprend une saillie 12a,12b configurée pour maintenir le tube souple 4 dans l'ouverture 3 de la pince.

En outre, comme montré sur les figures 5 et 6, le corps 2 de la pince comprend un évidement central 15a,15b. En particulier, la pince comprend sur chacune de ses faces supérieure et inférieure un évidement central 15a, 15b formant une bordure sur la périphérie du corps de la pince et autour de l'ouverture 3. Cette bordure possède une plus grande épaisseur que l'épaisseur du corps ainsi évidé. Cet évidement allège la pince.

La pince comprend en outre une partie de préhension 13 permettant à l'utilisateur de manipuler facilement la pince.

Dans cette troisième réalisation, cette partie de préhension 13 prolonge la base 10 du corps 2 de la pince dans le sens de la longueur. Avantageusement, la partie de préhension 13 comprend une pluralité de nervures 16a,16b. Les nervures sont parallèles entre elles et prévient le glissement de la pince entre les doigts de l'utilisateur. En particulier, chacune de la face supérieure et de la face inférieure de la partie de préhension 13 comprend une pluralité de nervures 16a,16b.

Selon les figures 5 et 6, la pince 2 comprend en outre un anneau 17 constitué d'un évidement de forme annulaire. Cet anneau est destiné à accrocher une étiquette d'identification de la pince.

La figure 7 est une vue de face de la pince selon cette troisième réalisation. Les surface de pincement 6,7 se rapprochent l'une de l'autre de sorte à réduire progressivement la section transversale du tube souple inséré dans cette pince, mais sans obturer ledit tube souple.

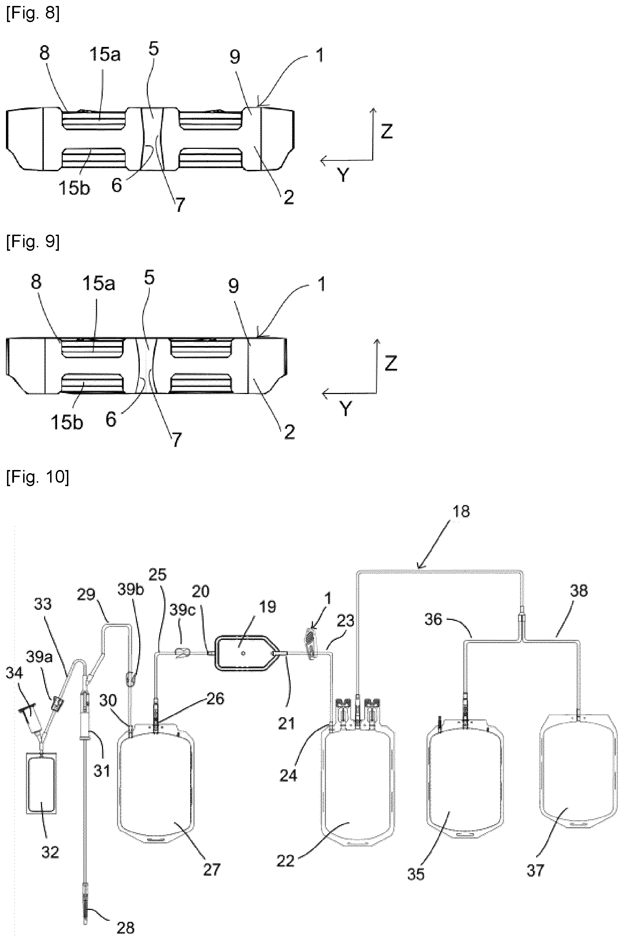

En variante représentée sur la figure 8, les surfaces de pincement 6,7 du corps de la pince se rapprochent l'une de l'autre avant de s'éloigner à nouveau l'une de l'autre. Dans cette variante, les surfaces de pincement sont substantiellement planes. Ainsi, la largeur de la portion de pincement au niveau de la surface supérieure et de la surface inférieur est la même, mais la largeur de la portion de pincement à environ la moitié de la hauteur de la portion de pincement est réduite.

Cette variante est avantageuse puisque la portion de pincement est symétrique par rapport à un plan passant par le milieu de la portion de pincement et perpendiculaire à l'axe z. Ainsi, quel que soit le sens de la pince lorsqu'on y insère le tube souple, la section transversale du tube souple comprimé sera la même avec d'abord une section transversale qui se réduit d'amont en aval avant de s'agrandir à nouveau. Dans cette variante, vue en coupe, les surfaces de pincement 6,7 forment substantiellement un sablier.

Par exemple, lorsque le tube souple possède un diamètre intérieur de 3 mm et un diamètre extérieur de 4,1 mm, la largeur du tube souple lorsqu'il est comprimé jusqu'à obturation est d'environ 5,1 mm. La hauteur de la portion de pincement est d'environ 5 mm. La portion de pincement possède par exemple une largeur d'environ 1,5 mm au niveau de la face supérieure et de la face inférieur. La largeur de la portion de pincement à environ 2,5 mm de hauteur est d'environ 1,3 mm. La longueur de la portion de pincement est d'environ 10 mm.

La figure 9 représente une deuxième variante de la troisième réalisation de la pince. Dans cette variante, la première et la deuxième surface de pincement sont substantiellement convexes. Ainsi, les surfaces de pincement 6,7 du corps de la pince se rapprochent l'une de l'autre avant de s'éloigner à nouveau l'une de l'autre. Cette forme convexe facilite l'insertion du tube souple dans la pince.

Selon un aspect, l'invention concerne un système pour réduire le débit d'un fluide dans un tube souple 4 comprenant une pince telle que décrite ci-dessus et ledit tube souple 4.

En particulier, le système comprend un tube souple 4 inséré dans l'ouverture 3 de la pince 1, au niveau de la portion de pincement 5 ou de la portion de maintien 14.

En faisant glisser le tube souple de la portion de maintien 14 vers la portion de pincement 5 de la pince 1, la section transversale du tube souple diminue, réduisant ainsi le débit du fluide dans le tube souple.

Selon un autre aspect, l'invention concerne un dispositif pour éliminer les leucocytes du sang ou d'un composant sanguin, comprenant d'une part un filtre pour éliminer les leucocytes du sang ou d'un composant sanguin et un premier tube souple en communication fluidique avec ledit filtre et d'autre part une pince selon le premier aspect de l'invention placée ou destinée à être placée sur le premier tube souple afin de réduire le débit dans ledit premier tube souple.

La figure 10 représente un tel dispositif 18 pour éliminer les leucocytes du sang ou d'un composant sanguin comprenant une pince 1 selon l'invention.

Le dispositif 18 comprend un filtre à éliminer les leucocytes 19 ayant un orifice d'entrée 20 et un orifice de sortie 21. Ledit filtre pour éliminer les leucocytes 19 est en communication fluidique avec une poche de recueil du filtrat 22 par l'intermédiaire d'un premier tube souple 23 dont l'une des extrémités est connectée à l'orifice de sortie 21 du filtre 19 et l'autre extrémité à un orifice d'entrée 24 de la poche de recueil du filtrat 22.

Le dispositif 18 comprend en outre un deuxième tube souple 25 dont l'une des extrémités est connectée à l'orifice d'entrée 20 du filtre pour éliminer les leucocytes 19 et l'autre extrémité est connectée ou destinée à être connectée à un orifice de sortie 26 d'une poche de collecte 27 contenant ou destinée à contenir le sang ou le composant sanguin à filtrer.

Dans la réalisation du dispositif de la figure 10, la poche de collecte 27 comprend une solution anticoagulante de type CPD (Citrate, Phosphate, Dextrose).

La pince 1 pour réduire le débit d'un fluide est placée ou destinée à être placée sur le premier ou deuxième tube souple 23,25. Avantageusement, la pince est placée sur le premier tube souple 23 en aval du filtre 19 pour éliminer les leucocytes. Par exemple, la pince 1 est placée sur le premier tube souple 23 à environ 5 cm en dessous de l'orifice de sortie 21 de filtre 19.

Selon la figure 10, le dispositif comprend en outre une aiguille de prélèvement 28 en communication fluidique par l'intermédiaire d'un troisième tube souple 29, avec un orifice d'entrée 30 de la poche de collecte 27 destinée à contenir le sang à filtrer. L'aiguille de prélèvement 28 est protégée par un capuchon et le troisième tube souple 29 comprend un protecteur d'aiguille 31 qui coulisse le long du troisième tube souple 29 jusqu'à recouvrir l'aiguille après le prélèvement de sang.

Le dispositif 18 comprend en outre une poche d'échantillonnage 32 du sang en communication fluidique avec l'aiguille de prélèvement, par l'intermédiaire d'un quatrième tube souple 33 branché sur le troisième tube souple 29. Un porte-tube 34 est en outre relié à la poche d'échantillonnage 32.

Le dispositif 18 comprend également une première poche satellite 35 en communication fluidique avec la poche de recueil du filtrat 22 par l'intermédiaire d'un cinquième tube souple 36. Cette première poche satellite 35 est destinée à recevoir le plasma séparé du sang après centrifugation du dispositif.

Une deuxième poche satellite 37 est en communication fluidique avec la poche de recueil du filtrat 22 par l'intermédiaire d'un sixième tube souple 38 branché sur le cinquième tube souple 36. Cette deuxième poche satellite 37 comprend une solution de conservation des globules rouges de type SAGM (Sodium, Adénine, Glucose, Mannitol).

Le dispositif 18 comprend en outre des clamps 39a,39b,39c pour contrôler l'écoulement des fluides dans le dispositif 18.

Un exemple d'utilisation du dispositif de la figure 10 est décrit ci-dessous.

On prélève d'abord le sang d'un donneur dans la poche de collecte 27 destinée à contenir le sang à filtrer par l'intermédiaire de l'aiguille de prélèvement 28.

On comprime le premier tube souple 23 à l'aide de la pince 1 selon l'invention. En variante, cette étape compression est réalisée avant le prélèvement du sang.

On transfère le sang contenu dans la poche de collecte 27 contenant le sang à filtrer dans la poche de recueil du filtrat 22 en le faisant passer au travers du filtre 19 pour éliminer les leucocytes. La poche de recueil du filtrat 22 contient alors le sang déleucocyté.

On place le dispositif 18 dans une centrifugeuse de sorte à séparer le sang déleucocyté contenu dans la poche de recueil du filtrat 22 en un sédiment de concentré de globules rouges et un surnageant de plasma.

On transfère le plasma dans la première poche satellite 35 puis la solution de conservation des globules rouges de la deuxième poche satellite 37 dans la poche de recueil du filtrat 22 contenant le concentré de globules rouges.

Dans un exemple particulier, le filtre 19 pour éliminer les leucocytes est le filtre LXT (Macopharma, France). Le dispositif 18 est ainsi apte à éliminer les leucocytes d'un sang total ayant une température comprise allant de 18 à 23°C sans utilisation de la pince et de 24° à 30°C avec utilisation de la pince.

### Exemple 1

Des essais de déleucocytation du sang total ont été réalisés à l'aide d'un système à poches du type de celui de la figure 10 comprenant un filtre à déleucocyter le sang total LXT (Macopharma, France) et d'une pince du type de celui de la figure 1.

La pince comporte un écartement de 1,5 mm d'un côté et 1,3 mm de l'autre et possède une épaisseur de 5 mm. Le tube souple en amont et le tube souple en aval du filtre LXT possèdent un diamètre extérieur de 4,1 mm et un diamètre intérieur de 3 mm.

Du sang total a été collecté dans les systèmes à poches mentionnés ci-dessus. Les poches contenant le sang total sont reçues un jour après le prélèvement et contiennent environ 475 ml de sang. Elles sont placées pendant une heure dans un bain marie à 30°C.

La filtration du sang total est réalisée dans 3 conditions différentes : sans pince, avec une pince placée au-dessus du filtre ou avec une pince placée en-dessous du filtre.

Les résultats obtenus sont les suivants :

**[Tableau 1]**

| | Sans pince | Pince au-dessus du filtre | Pince en-dessous du filtre |
|---|---|---|---|
| Nombre de filtration | 8 | 7 | 5 |
| Temps moyen de filtration (mm:ss) | 19:52 | 34:34 | 36:24 |
| Nombre moyen de globules blancs résiduels | 3,24x10⁵ | 2,51 x10⁵ | 1,47 x10⁵ |

La présence d'une pince au-dessus ou en-dessous du filtre à déleucocyter permet de réduire le débit du sang dans le filtre et conduit à une amélioration du taux de déleucocytation sans impact sur la perte de sang dans le filtre.

Après filtration, le sang total déleucocyté est séparé par centrifugation en concentré de globules rouges et en plasma.

La présence de la pince n'affecte pas le taux d'hémolyse dans le concentré de globules rouges ni la quantité de fibrinogène dans le plasma. Une légère baisse de la quantité de facteur VIII et de facteur XI dans le plasma est observée.

### Exemple 2

Des essais de déleucocytation ont été réalisés comme dans l'exemple 1 à l'aide de cinq pinces du type de celui de la figure 8.

Chaque pince comporte un écartement de 1,5 mm de chaque côté et un écartement de 1,3 mm au milieu. Elle possède une épaisseur de 5 mm. Le tube souple en amont et le tube souple en aval du filtre LXT possèdent un diamètre extérieur de 4,1 mm et un diamètre intérieur de 3 mm.

La pince est placée sur le tube souple en aval du filtre LXT.

Les résultats sont les suivants :

**[Tableau 2]**

| Pince | Temps de filtration (h:mm:ss) | Nombre de globules blancs résiduels (10⁶/poche) | Hémolyse (%) | Hémoglobine (g/poche) |
|---|---|---|---|---|
| Pince 1 | 00:25:31 | 0,10 | 0,01 | 51,0 |
| Pince 2 | 00:25:48 | 0,20 | 0,00 | 53,4 |
| Pince 3 | 00:26:20 | 0,73 | 0,15 | 65,1 |
| Pince 4 | 00:23:15 | 0,30 | 0,02 | 59,6 |
| Pince 5 | 00:19:28 | 0,36 | 0,00 | 49,3 |
| Pince 6 | 00:21:01 | 0,32 | 0,00 | 61,3 |
| Pince 7 | 00:20:40 | 0,09 | 0,00 | 40,6 |

On constate que le nombre de globules blancs résiduels est inférieur à 1. 10⁶ par poche, le taux d'hémoglobine est supérieur à 40 g par poche, le taux d'hémolyse est inférieur à 0,8%. Le produit sanguin est ainsi conforme aux normes européennes.

## Revendications

1. Système pour réduire le débit d'un fluide dans un tube souple (4) comprenant ledit tube souple (4) et une pince (1), ladite pince (1) comprenant un corps (2) pourvu d'une ouverture (3) destinée à recevoir ledit tube souple (4), l'ouverture ayant au moins une portion de pincement (5) configurée pour comprimer ledit tube souple (4) sans l'obturer, ladite portion de pincement (5) étant définie entre une première surface de pincement (6) et une deuxième surface de pincement (7) qui se font face, **caractérisé en ce que** lesdites surfaces de pincement (6,7) de la pince (1) se rapprochent l'une de l'autre sur une hauteur (h) plus petite ou égale à la largeur du tube souple lorsque ledit tube souple est comprimé jusqu'à obturation, de sorte à réduire le débit de fluide dans ledit tube souple, c'est-à-dire la distance entre les dites surfaces de pincement (6,7) est plus petite au niveau de la face inférieure de la pince qu'au niveau de la face supérieure.

2. Système selon la revendication 1, **caractérisé en ce que** l'ouverture (3) comprend en outre une portion d'insertion (11) configurée pour introduire le tube souple (4) dans ladite ouverture (3), ladite portion d'insertion (11) étant en communication avec la portion de pincement (5).

3. Système selon la revendication 1 ou 2, **caractérisé en ce que** l'ouverture (3) comprend en outre une portion de maintien (14) dans laquelle le tube souple (4) est maintenu sans être déformé, ladite portion de maintien (14) étant en communication avec la portion de pincement (5).

4. Système selon l'une quelconque des revendications 1 à 3, **caractérisé en ce que** la première et la deuxième surface de pincement (6,7) sont planes.

5. Système selon l'une quelconque des revendications 1 à 3, **caractérisé en ce que** la première et la deuxième surface de pincement (6,7) sont convexes.

6. Système selon l'une quelconque des revendications 1 à 5, **caractérisé en ce que** le corps de la pince possède un premier bras (8) et un deuxième bras (9) connectés entre eux par une base (10).

7. Système selon l'une quelconque des revendications 1 à 6, **caractérisé en ce que** le corps (2) de la pince comprend une partie de préhension (13) pourvue d'une pluralité de nervures (16a,16b).

8. Système selon l'une quelconque des revendications 1 à 7, **caractérisé en ce que** le corps (2) de la pince comprend un évidement central (15a, 15b).

9. Dispositif (18) pour éliminer les leucocytes du sang ou d'un composant sanguin, comprenant d'une part un filtre (19) pour éliminer les leucocytes du sang ou d'un composant sanguin et un premier tube souple (23) en communication fluidique avec ledit filtre (19) et d'autre part une pince (1) placée ou destinée à être placée sur le premier tube souple afin de réduire le débit dans ledit premier tube souple, ledit premier tube souple (23) et ladite pince (1) formant un système selon l'une quelconque des revendications 1 à 8.

10. Dispositif selon la revendication 9, **caractérisé en ce que** l'une des extrémités du premier tube souple (23) est connectée à un orifice de sortie (21) dudit filtre (19) pour éliminer les leucocytes et l'autre extrémité est connectée à un orifice d'entrée (24) d'une poche de recueil du filtrat (22).

## Patentansprüche

1. System zur Reduzieren der Strömung eines Fluids in einem flexiblen Rohr (4), umfassend das flexible Rohr (4) und eine Klemme (1), wobei die Klemme (1) einen Körper (2) umfasst, der mit einer Öffnung (3) versehen ist, die dazu bestimmt ist, das flexible Rohr (4) aufzunehmen, wobei die Öffnung mindestens einen Klemmabschnitt (5) aufweist, der konfiguriert ist, um das flexible Rohr (4) zusammenzudrücken, ohne es zu verschließen, wobei der Klemmabschnitt (5) zwischen einer ersten Klemmfläche (6) und einer zweiten Klemmfläche (7) definiert ist, die einander zugewandt sind, **dadurch gekennzeichnet, dass** die Klemmflächen (6,7) der Klemme (1) sich einander um eine Höhe (h) annähern, die kleiner als oder gleich wie die Breite des flexiblen Rohrs ist, wenn das flexible Rohr bis zum Verschluss zusammengedrückt wird, um die Fluidströmung durch das flexible Rohr zu verringern, das heißt der Abstand zwischen den Klemmflächen (6,7) ist an der Unterseite der Klemme kleiner als an der Oberseite.

2. System nach Anspruch 1, **dadurch gekennzeichnet, dass** die Öffnung (3) ferner einen Einführabschnitt (11) umfasst, der konfiguriert ist, um das flexible Rohr (4) in die Öffnung (3) einzuführen, wobei der Einführabschnitt (11) mit dem Klemmabschnitt (5) in Verbindung ist.

3. System nach Anspruch 1 oder 2, **dadurch gekennzeichnet, dass** die Öffnung (3) ferner einen Halteabschnitt (14) umfasst, in dem das flexible Rohr (4) gehalten wird, ohne verformt zu werden, wobei der Halteabschnitt (14) mit dem Klemmabschnitt (5) in Verbindung ist.

4. System nach einem der Ansprüche 1 bis 3, **dadurch gekennzeichnet, dass** die erste und die zweite Klemmfläche (6,7) eben sind.

5. System nach einem der Ansprüche 1 bis 3, **dadurch gekennzeichnet, dass** die erste und die zweite Klemmfläche (6,7) konvex sind.

6. System nach einem der Ansprüche 1 bis 5, **dadurch gekennzeichnet, dass** der Körper der Klemme einen ersten Arm (8) und einen zweiten Arm (9) aufweist, die durch eine Basis (10) miteinander verbunden sind.

7. System nach einem der Ansprüche 1 bis 6, **dadurch gekennzeichnet, dass** der Körper (2) der Klemme einen Greifabschnitt (13) umfasst, der mit einer Vielzahl von Rippen (16a,16b) versehen ist.

8. System nach einem der Ansprüche 1 bis 7, **dadurch gekennzeichnet, dass** der Körper (2) der Klemme eine mittlere Aussparung (15a,15b) umfasst.

9. Vorrichtung (18) zum Entfernen der Leukozyten aus Blut oder einer Blutkomponente, einerseits umfassend einen Filter (19) zum Entfernen der Leukozyten aus Blut oder einer Blutkomponente und ein erstes flexibles Rohr (23) in Fluidverbindung mit dem Filter (19) und andererseits einer Klemme (1), die auf dem ersten flexiblen Rohr platziert ist oder darauf platziert werden soll, um die Strömung durch das erste flexible Rohr zu reduzieren, wobei das erste flexible Rohr (23) und die Klemme (1) ein System nach einem der Ansprüche 1 bis 8 bilden.

10. Vorrichtung nach Anspruch 9, **dadurch gekennzeichnet, dass** ein Ende des ersten flexiblen Rohrs (23) mit einer Auslassöffnung (21) des Filters (19) verbunden ist, um die Leukozyten zu entfernen, und das andere Ende mit einer Einlassöffnung (24) eines Filtratsammelbeutels (22) verbunden ist.

## Claims

1. System for reducing the flow rate of a fluid in a flexible tube (4) comprising said flexible tube (4) and a clamp (1), said clamp (1) comprising a body (2) provided with an opening (3) intended for receiving said flexible tube (4), the opening having at least one pinching portion (5) configured to compress said flexible tube (4) without obstructing it, said pinching portion (5) being defined between a first pinching surface (6) and a second pinching surface (7) which face each other, **characterized in that** said pinching surfaces (6, 7) of the clamp (1) move closer to each other over a height (h) less than or equal to the width of the flexible tube when said flexible tube is compressed until obstruction thereof, so as to reduce the fluid flow rate in said flexible tube, i.e., the distance between said pinching surfaces (6, 7) is smaller at the lower face of the clamp than at the upper face.

2. System according to claim 1, **characterized in that** the opening (3) further comprises an insertion portion (11) configured to introduce the flexible tube (4) into said opening (3), said insertion portion (11) being in communication with the pinching portion (5).

3. System according to claim 1 or 2, **characterized in that** the opening (3) further comprises a holding portion (14) in which the flexible tube (4) is held without being deformed, said holding portion (14) being in communication with the pinching portion (5).

4. System according to any of claims 1 to 3, **characterized in that** the first and second pinching surfaces (6, 7) are flat.

5. System according to any of claims 1 to 3, **characterized in that** the first and second pinching surfaces (6, 7) are convex.

6. System according to any of claims 1 to 5, **characterized in that** the body of the clamp has a first arm (8) and a second arm (9) connected to each other by a base (10).

7. System according to any one of claims 1 to 6, **characterized in that** the body (2) of the clamp comprises a gripping portion (13) provided with a plurality of ribs (16a, 16b).

8. System according to any of claims 1 to 7, **characterized in that** the body (2) of the clamp comprises a central recess (15a, 15b).

9. Device (18) for removing leukocytes from blood or a blood component, comprising, on the one hand, a filter (19) for removing leukocytes from blood or a blood component and a first flexible tube (23) in fluid communication with said filter (19) and, on the other hand, a clamp (1) placed or intended to be placed on the first flexible tube in order to reduce the flow rate in said first flexible tube, said first flexible tube (23) and said clamp (1) forming a system according to any of claims 1 to 8.

10. Device according to claim 9, **characterized in that** one end of the first flexible tube (23) is connected to an outlet port (21) of said filter (19) for removing leukocytes and the other end is connected to an inlet port (24) of a filtrate collection bag (22).
